Europäisches Patentamt

European Patent Office

Office européen des brevets

(19)

(11) Publication number: **0 099 167**

**A1**

(12) # EUROPEAN PATENT APPLICATION

(21) Application number: **83302001.9**

(22) Date of filing: **08.04.83**

(51) Int. Cl.³: **A 61 F 1/03**

(30) Priority: **14.05.82 US 378103**

(43) Date of publication of application:
**25.01.84 Bulletin 84/4**

(84) Designated Contracting States:
**CH DE FR GB LI**

(71) Applicant: **Carbomedics Inc.**
**1300 East Anderson Lane**
**Austin Texas(US)**

(72) Inventor: **Yapp, Ronald Arthur**
**9012 W. Pointer Lane**
**Austin Texas(US)**

(74) Representative: **Barnard, Eric Edward et al,**
**BROOKES & MARTIN High Holborn House 52/54 High**
**Holborn**
**London WC1V 6SE(GB)**

(54) Proximal femur implant.

(57) A femur implant for a hip prosthesis has a sleeve (12) adapted for insertion and fixation by ankylosis within a passageway that is cut to extend generally linearly through the femur from a prepared neck cut to the lateral cortex. The sleeve (12) has a shaft portion (13) of rectangular cross section insertable into the passageway and a flange (24) which seats against the prepared neck cut, the shaft (13) and flange (24) having porous bone-contact surfaces (20, 22) which promote bone tissue ingrowth. The passageway is precisely cut to match the configuration of the shaft (13), and a threaded bolt (28) and washer (47) pull the sleeve (12) toward the lateral side of the femur to hold the porous surfaces (20, 22) in close contact with the prepared bone surfaces during healing.

EP 0 099 167 A1

Croydon Printing Company Ltd.

## PROXIMAL FEMUR IMPLANT

The present invention relates to hip joint prostheses and more particularly to improved femoral implants for hip prostheses.

A variety of hip joint prostheses for replacement of the natural hip joint have been proposed and used.  In a hip joint replacement, the defective natural ball and neck of the femur are surgically removed and replaced with an artificial ball and neck. The artificial ball or head may be shaped to fit in the natural acetabulum (pelvic socket), or if the natural acetabulum is defective, a synthetic socket for receiving the artificial head may be surgically implanted in the pelvis.

Implanted hip joints generally derive a major portion of their support from elongated stems which are inserted into surgically reamed bores through the medullary cavities.  The stems may be supported within the medullary cavities by contact with the cortical surfaces and in some cases, such as in Baumann et al. Patent No. 4,101,985, by pins extending through the lateral cortex.  Generally, a cement is used to fill the cavity around an inserted stem in order to provide rapid fixation of the stem in the bone.

Due to a variety of causes, hip joint prostheses have not yet achieved the success rate which would be desirable.  Although metals used to form support stems are generally stronger than the natural bone tissue, metal failure has been responsible for a number of hip prosthesis failures.  If metal failure does not occur, the decrease in stress on the bone created by the inserted stem may cause stress by-pass ostepenia, i.e., localized bone degeneration, to occur. The stress may also crack the cement resulting in a loose fit of the stem within the medullary cavity and higher stresses in the stem and potential fatigue failure of the stem.

In order to eliminate the need to use cement, it has been proposed to use stems coated with porous metal, such as porous cobalt-chromium or titanium alloys which promote tissue ingrowth and fixation of the stem to the bone; however, the potential for a stress by-pass problem to occur still exists and may be even greater when the stem is well fixed to the bone. Also, the success of such implants (arthroplasties), which are joined to the bone by ankylosis, depends on rapid fixation of a relatively immobile implant through tissue ingrowth. In addition the reaming and broaching of the bone needed to provide a passageway for the long medullary stems compromises the blood supply necessary for normal tissue growth and implant fixation. Hence, the need continues for improved hip prostheses.

A femur implant for a hip prosthesis has a sleeve designed for tissue ingrowth fixation adjacent to the transected surface and within a passageway that is cut to extend generally linearly through the femur from a prepared neck cut to the lateral cortex. The sleeve has a shaft insertable into the passageway and a flange which seats against the prepared neck cut, the shaft and flange having porous bone-contact surfaces which promote tissue ingrowth. The passageway is precisely cut to match the configuration of the shaft, and a threaded member pulls the sleeve toward the lateral side of the femur to immobilize and hold the porous surfaces in close contact with the prepared bone surfaces during the implant fixation period.

FIGURE 1 is an exploded perspective view of a femur implant assembly for a hip joint prosthesis embodying various features of the present invention;

FIGURE 2 is an elevation view of the partially assembled femur implant;

FIGURE 3 is an enlarged perspective view of the sleeve of the implant;

FIGURE 4 is an elevation view, partially in cross section, of the sleeve of FIGURE 3;

FIGURE 5 is an end elevation view of the sleeve;

FIGURE 6 is a perspective view of the femur in which the assembly has been surgically implanted; and

FIGURE 7 is an enlarged cut away view of the upper end of the femur having the implant therein.

In accordance with the present invention, a femur implant portion, indicated generally at 10, of a hip joint prosthesis has a sleeve 12 including a shaft 13 that extends into a passageway 14 (FIG. 7) formed generally linearly from a surgically transected neck surface 16 of a femur 18 across the femur and through the lateral medullary cortex 19. A flange 24 at an outer end 26 of the shaft 13 seats against the transected neck surface 16 of the femur 18, while a means 28 at the lateral side of the femur is attached to the inner end 30 of the shaft to hold the sleeve surfaces 20, 22 firmly against the bone surfaces of the passageway as healing is taking place. The sleeve 12 has porous bone contact surfaces 20, 22 which promote tissue (bony and/or fibrous) ingrowth and fixation of the sleeve within the femur 18.

As illustrated herein, the femur implant 10 is preferably formed as an assemblage of separate members with a neck 32 and head or ball 34 of the implant formed separately from the sleeve 12. This enables a greater variety of different length and size prostheses to be provided and enables the surgeon to select a neck and ball which best fit the patient subsequent to implanting the sleeve 12. The spacing of the femur from the pelvis, as provided by the length of the artifical neck 32, is critical to the success of the hip joint replacement, and the cutting of the femur neck has been of critical importance in prostheses having shafts integral with the neck. With a prosthesis provided as an assemblage of a separate sleeve 12, neck 32 and head 34, the surgeon can initially concentrate fully on implantation of the sleeve into the femur 18 and thereafter fit the neck and head according to the anatomy of the patient. It also eliminates the need of removing a fixated stem or sleeve when the ball or neck need to be changed due to a change in the condition of the acetabulum.

The illustrated sleeve 12 has a shaft 13, which has generally flat surfaces and rounded corners and edges that are proportioned for insertion into the passageway 14, formed to extend generally linearly from the prepared neck 16 of the femur 18 at an angle downward through the femur and emerge from the lateral cortex 19 just below the trochanter 38. A relatively wide passageway portion 40 is formed to extend from the medial side 42 of the femur, across the medullary cavity 44 to the lateral cortex 19. This wide passageway portion 40 preferably has a rectangular cross section that may decrease or taper somewhat towards its inner end. This passageway portion 40 is generally perpendicular to the generally flat transected surface 16 of the neck. Passageways through bone, of such configuration, may be cut with great precision by using specially designed fixturing and cutting instruments, and the passageway 14 is cut with a configuration matched to the shaft 13 so that the shaft will fit tightly therein. A much narrower bore portion 44 is drilled through the remainder of the lateral cortex 19. A broad, shallow depression 46 is rounded from the exterior of the lateral cortex 19 just below the trochanter 38 to provide good seating and ingrowth into the porous interior surface of a grommet or washer 47 as hereinafter described. In total, a relatively small amount of bone tissue is removed, and the blood supply needed to promote healing is only minimally disrupted.

The shaft 13, which is inserted into the enlarged portion 40 of the passageway 14, is generally in the shape of a truncated rectangular pyramid having a rectangular cross section with rounded corners and edges that narrows towards its inner end. When tension is applied to the sleeve 12 to pull it in the direction of the lateral cortex 19, the tapered side surfaces 20 of the shaft 13 abut against the side surfaces of the passageway that are cut with a complementary taper. The passageway 40 is formed so that the shaft 13 locates therein with two of its side surfaces 20 generally parallel and two oblique to the longitudinal axis of the femur 18, and at the region of insertion, i.e., in the transected surface 16 of the femur 18, the sleeve sides are predominantly perpendicular to the natural loading direction of the cortical bone they bear against. Simulating the natural loading stresses is a particularly important feature of an implant 10 which relies on tissue ingrowth for fixation. In contrast to a cylindrical surface, the flat shaft surfaces 20 more evenly distribute the load avoiding shear as much as possible.

The flange 24 at the outer end of the sleeve 12 limits the extent of shaft 13 insertion and helps to distribute the sleeve load axially into the cortex at the transected surface of the femoral neck 16. The flange 24 is considerably broader than the shaft 13, extending along most of the transected surface, and is generally flat to seat in good contact with the transected surface. The flange 24 angles from the surfaces 20 of the inserted portion 13 to correspond with the angles that the passageway sides angle from the flat transected surface 16. The lower end 50 of the flange 24 preferably is chamfered to seat against the medial cortex 42, generally perpendicular to the longitudinal femur axis, to exert loading forces downward through the medial cortex.

Along the transected surface 16 and within the passageway 14 which is precisely formed to complement the configuration of the shaft 13, the sleeve surfaces 20, 22 are in direct surface contact with the femur over substantial areas, i.e., along the shaft sides 20 and the inner surface 22 of the flange 24. The sleeve 12 is formed of a strong biocompatible metal, such as titanium or a cobalt-chromium alloy, or a polymer, such as graphite-reinforced epoxy, and the bone contact surfaces 20, 22 have a coating 52 (FIG. 4) of porous biocompatible metal, such as porous chrome-cobalt, or porous polymer or mineral (such as H.A.P.) which promotes tissue ingrowth into the bone-contact surfaces and tissue fixation of the sleeve to the femur.

To hold the shaft 13 within the passageway 40 under tension from the lateral side of the femur 18, a threaded bolt 28 is screwed into a threaded bore 60 in the inner end 30 of the shaft 13 with the grommet 47 serving as a washer. The grommet 47 is placed along the prepared shallow depression 46, and the bolt 28 is passed through a narrow orifice 70 in the grommet 47 and screwed into the threaded bore 60 in the shaft. When the bolt 28 is attached to the shaft 13 and tightened, the grommet 47 is pressed firmly into the depression 46 and tension is applied to the sleeve 12 pulling it toward the lateral side of the femur and pressing its bone contact surfaces 20, 22 into abutting contact against the cut bone tissue.

The grommet 47 has an inner surface 62 generally in the shape of a sector of a sphere, and the broad, shallow depression 46 is formed in the femur to a corresponding shape to provide a tight interface between the grommet and the cut bone. The inner surface 62 of the grommet, like the bone contact surfaces 20, 22 of the sleeve 12, has a porous coating 64 suitable for tissue ingrowth and implant fixation. The outer surface 66 of the grommet is frustoconical and has a depression 68 for countersinking the head 56 of the bolt 28 coaxial with the narrow orifice 70 through which the thread 48 of the bolt extends.

As a means of maintaining the tension between the shaft flange 24 and the grommet 47 that brings the shaft surfaces 22 in tight contact with the passageway sides, a spring member 72, such as a Bellville washer, is interposed between the bolt head 56 and the inner surface of the grommet depression 68. Tightening of the bolt 28 creates tension on the spring member 72 that acts to prevent loosening of the sleeve 12 within the femur 18 should any bone compression or wear or adjustment of the sleeve assembly occur during fixation.

The tension created by the bolt 28 upon tightening into the inner end 30 of the shaft 13 immobilizes the sleeve 12 during the time in which bone ingrowth occurs. When the ingrowth has progressed sufficiently, the sleeve and bone function as a unitary member which should never have to be replaced.

Providing the femur implant portion of a hip prosthesis as an assemblage does, however, contemplate that other portions of the prosthesis may have to be replaced. For example, if the natural femur head is defective but the pelvic socket is in good condition, a hemispherical head 34 might be used in conjunction with the neck 32 and sleeve 12 and inserted directly into the natural acetabulum.

While the head 34 should provide for long term functioning of the hip joint, experience has shown that the acetabulum may eventually be worn away by the artificial head of a femur implant. If excessive deterioration of the acetabulum occurs, another operation is performed on the patient in which the acetabulum is reamed out and replaced with an artificial pelvic socket, such as might be formed of polyethylene, which receives a spherical head of a femur implant. In hip prosthesis in which the neck, or neck and ball, of the femur implant are integral with the supporting shaft, replacement of the head generally requires replacement of the entire femur implant, including the shaft, thus entailing additional reaming of the bone and further weakening of the natural bone structure. When one considers that many hip replacements are performed on older patients with brittle bones, the desirability of never having to remove the implanted sleeve can be readily appreciated. The assemblage, in which the neck 32 and head 34 are formed separately from the sleeve 12, contemplates that the implanted and ankylosed sleeve will never be removed. If further operations are necessary to adapt the implant to fit into an artificial rather than the natural socket, the neck and head are removable and replaceable in the sleeve 12.

The head or ball 34 of the prosthesis has a surface which is a sector of a sphere which ranges from approximately one-half of a sphere to nearly a full sphere depending on the anatomy of the patient and whether the head is to be received in a natural or artificial socket. A flat outer surface 80 of the otherwise spherical head includes means 82 of connecting the head to the neck 32. The head is formed of a durable biocompatible material, such as chrome-cobalt or pyrolytic carbon, and may be coated with a durable low-friction coating, such as vapor-deposited graphite.

The neck 32 is a rod in the shape of a circular or elliptical cylinder having a terminal 84 at one end for connecting it to the sleeve 12 and a terminal 86 at its other end for connecting it to the head 34. The neck 32 is formed of a material, such as titanium, chromium-cobalt alloy, or graphite-reinforced polymer to approximate the stiffness of the natural neck. Unlike some current neck designs for femoral hip components, the construction of the neck 32 recognizes that the stiffness of the natural neck helps absorb some of the loading energy, and this stiffness should be duplicated as nearly as possible in the neck of a hip prosthesis, particularly in a prosthesis relying on tissue ingrowth for fixation. In this regard the dimensions and material are chosen to provide a stiffness (load vs. deflection) as determined from the natural femoral neck and strength such that fatigue is not a problem. The stiffness and strength may be graded to correspond to three or four patient weight ranges.

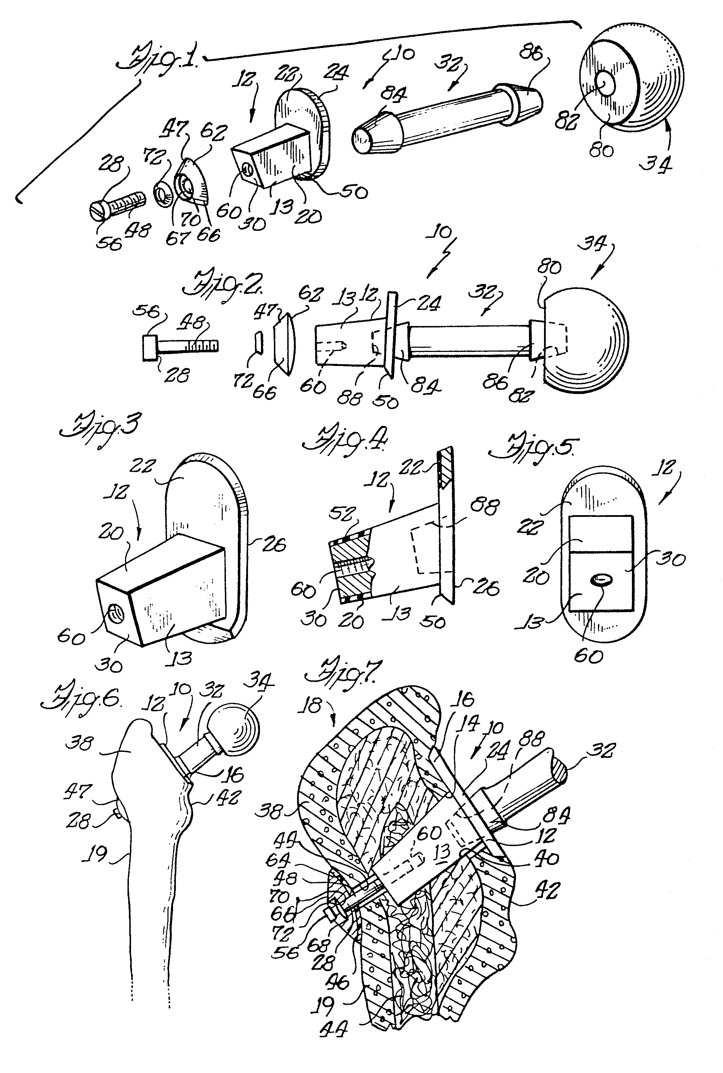

Locking tapers 82, 84, 86, 88 are preferred to join the neck 32 to the shaft and to the head 34. Female locking tapers 82, 88 respectively, extend radially into the flat outer surface 80 of the head 34 and into the outer end 26 of the sleeve 12, where it is preferably coaxial with the threaded bore 60 at the opposite end. As seen in FIG. 2, the sleeve, neck and head interfit to form a generally linear prosthesis. After the surgeon has implanted the shaft 12 and selected a head 34 and neck 32 to match the femur implant to the patient's anatomy, the shaft, neck and head are joined together by inserting the male tapers 84, 86 into the female tapers 82, 88 and lightly impacting them to effect a locked union of the assemblage. An appropriate adhesive may be used to additionally secure the assemblage but should not be necessary. If adhesive bonds are formed, they should be removable, e.g., through use of solvents or with appropriate surgical tools, should the need for replacement of the head ever arise.

The advantages of the invention should now be more fully apparent. The method and apparatus of the invention provide for insertion of an implant in a femur with a minimum of bone tissue and circulation loss. The implant is inserted in the proximal portions of the femur in the region of the thickened trochanter, and the configuration of the insert results in loading of the femur in this region generally along the natural lines of stress. The bone contact surfaces of the prosthesis are coated with a porous material that promotes tissue ingrowth so that implant fixation will occur whereby the sleeve eventually functions as a part of the femur. During the process of implant fixation, the sleeve is held under tension within the formed passageway immobilizing the sleeve and holding the shaft surfaces in tight immobilized interface with the surfaces of the passageway. By fixing the prosthesis to the bone by tissue ingrowth rather than cement, the problem of cracking cement is avoided, and by eliminating the use of a shaft or stem down through the medullary cavity, related stress problems are avoided.

While the invention has been described in terms of a preferred embodiment, modifications obvious to one with ordinary skill in the art may be made without departing from the scope of the invention. For example, while it is preferred that the prosthesis be formed as an assemblage of shaft, neck and head, these components or combinations of these components might be formed integrally. Instead of a bolt extending into the femur from the lateral side of the femur and attaching to the shaft, the shaft might have an integral threaded stud which emerges from the lateral end of the passageway to which the grommet and a nut are applied.

Various features of the invention are set forth in the following claims.

0099167

-1-

CLAIMS:

1. A femur implant insertable in a passageway formed to extend generally perpendicularly from a surgically transected neck surface of the femur at the medial side of the trochanter through to the lateral medullary cortex, the prosthesis comprising a head adapted for rotation within a natural or artificial pelvic socket; a neck having a portion at one end to interconnect with said head; a sleeve having a shaft portion insertable into the passageway from the transected neck surface proportioned for substantial contact with the bone surfaces of the passageway, a flange at an outer end of said shaft portion broader than said shaft portion and proportioned to seat against the transected neck surface when said shaft portion is inserted into the passageway, means for interconnecting the flange end of said shaft portion with the other end of said neck, and porous bone-contact surfaces on said shaft portion and said flange for promoting tissue ingrowth and implant fixation of said prosthesis within the femur; and means positionable along the lateral side of the femur and attachable to said sleeve for pulling said shaft toward the lateral side, whereby said sleeve is held in abutting contact against surfaces of the bone passageway during fixation.

2. An implant according to Claim 1 wherein said sleeve, neck and head are individually fabricated members.

3. An implant according to Claim 2 wherein said portions that interconnect said neck to said head and said sleeve to said neck have locking tapers.

4. An implant according to Claim 1 including a grommet having a porous bone-contact surface, said pulling means holding said grommet in contact with the lateral side of the femur.

5. An implant according to Claim 1 wherein the surfaces of said shaft are flat to complement flat surfaces of a cut femur passageway that is generally linear in the trans-femur direction.

6. An implant according to Claim 1 wherein said shaft has a generally rectangular cross section and flat bone-contact surfaces.

7. An implant according to Claim 1 wherein said flange has a surface adapted to contact the medial cortex of the femur perpendicular to the longitudinal axis of the femur.

8. An implant according to Claim 1 wherein said neck has a stiffness generally equal to the stiffness of the replaced natural femur neck.

9. A method of implanting hip prosthesis sleeve in a femur comprising cutting the neck of a defective femur, providing a prosthesis having a sleeve with a generally linear shaft, a broad outer flange for seating along the cut neck, and porous bone contact surfaces to be advantageous to tissue ingrowth, forming a passageway having a configuration matched to said shaft from the neck cut generally linearly through the femur to emerge from the lateral cortex below the trochanter, inserting said shaft into said passageway until said flange seats along the cut neck, and attaching a member from the lateral side of the femur to said sleeve to hold said shaft under tension within said passageway during the tissue ingrowth period.

10. A method according to Claim 9 including holding a grommet having a porous bone contact-surface against the lateral cortex of the femur with said member.

11. A method according to Claim 10 including forming a broad shallow depression in the lateral cortex to receive said grommet.

12. A method according to Claim 9 including interposing biasing means between the lateral cortex and said member to maintain continuous tension on said sleeve pulling it in the direction of the lateral cortex.

13. A method according to Claim 9 using a sleeve having means for connecting the same to a separately formed head and neck.

Fig.1.

Fig.2.

Fig.3

Fig.4.

Fig.5.

Fig.6.

Fig.7.

# 0099167

**European Patent Office**

**PARTIAL EUROPEAN SEARCH REPORT**
which under Rule 45 of the European Patent Convention shall be considered, for the purposes of subsequent proceedings, as the European search report

Application number

## DOCUMENTS CONSIDERED TO BE RELEVANT

EP 83302001.9

| Category | Citation of document with indication, where appropriate, of relevant passages | Relevant to claim | CLASSIFICATION OF THE APPLICATION (Int. Cl. ³) |
|---|---|---|---|
| X,Y | GB - A - 2 033 755 (A. RAMBERT)<br>* Totality * | 1,2,3,8 | A 61 F 1/03 |
| Y | FR - A1 - 2 429 009 (CH. ROUX)<br>* Fig. 5; page 6, lines 13-18 * | 1,2,3,8 | |
| A | GB - A - 1 579 769 (A. HUGGLER)<br>* Fig. 2; page 2, line 94 - page 3, line 65 * | 1,7,8 | |
| D | US - A - 4 101 985 (F. BAUMANN et al.) | | |
| A | * Fig. 7; column 4, lines 35-46 * | | |

---

TECHNICAL FIELDS
SEARCHED (Int. Cl ³)

A 61 F 1/00

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely: 1-8
Claims searched incompletely: —
Claims not searched: 9-13
Reason for the limitation of the search: in order to Art. 52(4)

| Place of search | Date of completion of the search | Examiner |
|---|---|---|
| VIENNA | 07-09-1983 | LUDWIG |